# EUROPEAN PATENT APPLICATION

(11) **EP 4 848 296 A1**
(43) Date of publication of application: **02.12.2026**
(21) Application number: 24920159.1
(22) Date of filing: 09.12.2024
(51) Int. Cl.: G16H 20/00, G16H 50/30

(54) **CALCULATION SYSTEM, CALCULATION METHOD, AND CALCULATION PROGRAM**

(30) Priority: 25.01.2024 JP 2024009325
(71) Applicant: Noster Inc., Muko-shi, Kyoto 617-0006 (JP)
(72) Inventor: KITAO, Kohey, Muko-shi, Kyoto 6170006 (JP)
(74) Representative: Hoffmann Eitle
(86) International application number: PCT/JP2024/043425
(87) International publication number: WO 2025/158802

(57) **Abstract**

A risk of contracting a specific disease is calculated. A calculation system includes: a metabolite data acquisition unit configured to acquire subject metabolite data relating to amounts or concentrations of a plurality of metabolites in a subject; a metabolite data extraction unit configured to extract, from the subject metabolite data that have been acquired, subject metabolite data of at least one metabolite corresponding to a target disease for which a score is to be calculated, on the basis of correspondence between metabolites and diseases; a score calculation unit configured to calculate a disease score relating to a risk of the target disease in the subject on the basis of statistical association between the subject metabolite data that have been extracted and a reference individual data group that is acquired in advance and includes reference individual metabolite data relating to an amount or concentration of the at least one metabolite in a plurality of reference individuals; and a score output unit configured to output the disease score.

## Description

### Technical Field

This application is based on Japanese Patent Application No. 2024-009325, filed on 25 January 2024, the contents of which are incorporated herein by reference.

The present disclosure relates to a calculation system, a calculation method, and a calculation program.

### Background Art

Conventionally, techniques for analyzing biological samples obtained from subjects are known. For example, Patent Literature 1 describes an information processing system that includes an intestinal environment data acquisition unit that acquires intestinal environment data of a user, an intestinal environment evaluation unit that evaluates the user's intestinal environment on the basis of the acquired intestinal environment data, and an information provision unit that provides the user with information including evaluation.

### Citation List

### Patent Literature

Patent Literature 1: WO 2021/153574

### Summary of Invention

### Technical Problem

The information processing system described in Patent Literature 1 provides subjects with information relating to the tendencies of dietary habits of the subjects. As such, although the subjects can recognize the tendencies of their own dietary habits, the subjects cannot recognize risks of contracting specific diseases.

### Solution to Problem

Accordingly, in this disclosure, it is an object of the present disclosure to calculate a risk of contracting a specific disease.

A calculation system according to one aspect of the present disclosure includes: a metabolite data acquisition unit configured to acquire subject metabolite data relating to amounts or concentrations of a plurality of metabolites in a subject; a metabolite data extraction unit configured to extract, from the subject metabolite data that have been acquired, subject metabolite data of at least one metabolite corresponding to a target disease for which a score is to be calculated, on the basis of correspondence between metabolites and diseases; a score calculation unit configured to calculate a disease score relating to a risk of the target disease in the subject on the basis of statistical association between the subject metabolite data that have been extracted and a reference individual data group that is acquired in advance and includes reference individual metabolite data relating to an amount or concentration of the at least one metabolite in a plurality of reference individuals; and a score output unit configured to output the disease score.

According to the present disclosure, a risk of contracting a specific disease can be calculated.

### Brief Description of Drawings

[Figure 1] Figure 1 is a diagram schematically showing processing of a calculation system 100, which is one embodiment of the present disclosure.
[Figure 2] Figure 2 is a diagram showing the configuration of a calculation system 100, which is one embodiment of the present disclosure.
[Figure 3] Figure 3 is a diagram showing an example of correspondence data stored in a storage unit 110.
[Figure 4] Figure 4 is a diagram showing an example of a reference individual data group stored in the storage unit 110.
[Figure 5] Figure 5 is a diagram showing an example of subject metabolite data stored in the storage unit 110.
[Figure 6] Figure 6 is a diagram showing an example of message templates stored in the storage unit 110.
[Figure 7] Figure 7 is a diagram showing an example of a report output by the calculation system 100.
[Figure 8] Figure 8 is a diagram showing an example of a report output by the calculation system 100.
[Figure 9] Figure 9 is a diagram showing an example of a report output by the calculation system 100.
[Figure 10] Figure 10 is a flowchart showing an example of processing of the calculation system 100.
[Figure 11] Figure 11 is a diagram showing an example of the hardware configuration of a computer 1100.

### Description of Embodiments

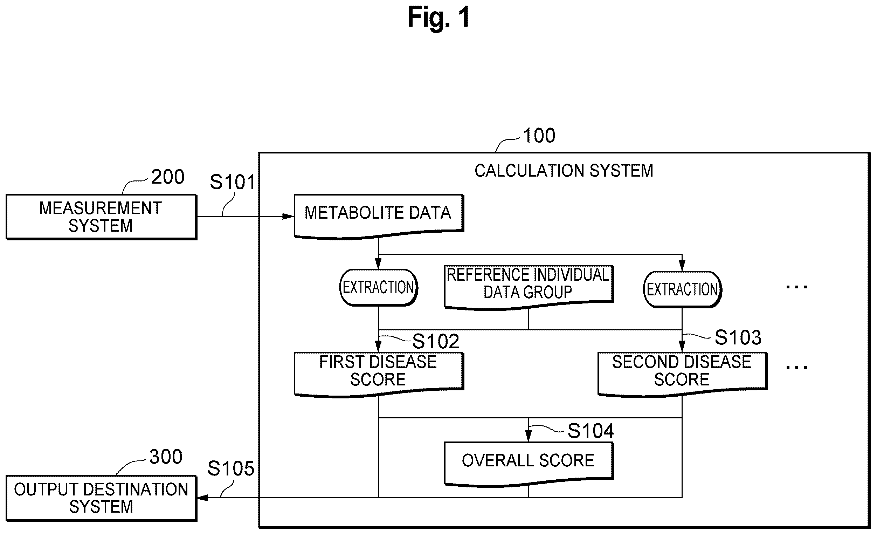

Preferred embodiments of the present disclosure are described with reference to the attached drawings. Figure 1 is a diagram schematically showing processing of a calculation system 100, which is one embodiment of the present disclosure.

The calculation system 100 is an information processing system implemented by a calculation program. The calculation system 100 is an information processing system that calculates and outputs, on the basis of subject metabolite data of a subject and a reference individual data group, a disease score relating to a risk of a disease in the subject and an overall score based on a plurality of disease scores.

First, the calculation system 100 obtains subject metabolite data of a subject from a measurement system 200 (S101). The calculation system 100 extracts, of the acquired subject metabolite data, subject metabolite data of at least one metabolite corresponding to a target disease for which a score is to be calculated, and calculates a first disease score on the basis of at least one piece of extracted subject metabolite data and a reference individual data group (S102). The calculation system 100 also calculates a disease score for another target disease (for example, a second disease score) in a similar manner (S103).

The calculation system 100 then calculates an overall score on the basis of multiple disease scores (for example, the first disease score and the second disease score) (S104). The calculation system 100 outputs the multiple disease scores (for example, the first disease score and the second disease score) and the overall score to an output destination system 300.

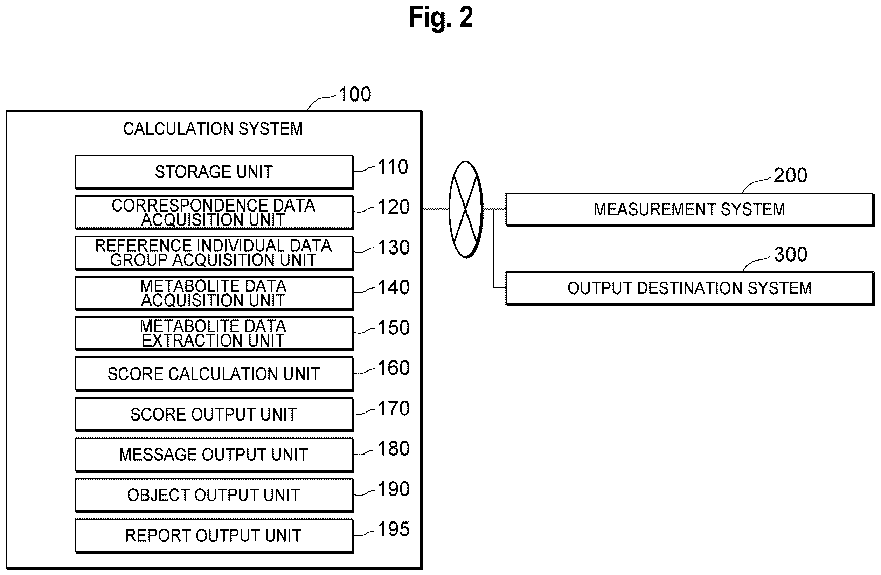

Figure 2 is a diagram showing the configuration of a calculation system 100, which is one embodiment of the present disclosure. The calculation system 100 is communicatively connected to a measurement system 200 and an output destination system 300 via a network such as the Internet. Details of the calculation system 100 are described below. The calculation system 100 and the measurement system 200 may exchange information via an interface such as a universal serial bus (USB) instead of via a network such as the Internet.

The measurement system 200 is an information processing system that measures subject metabolite data relating to a plurality of metabolites of a subject.

For example, the measurement system 200 obtains a predetermined sample (e.g., feces, urine, blood, saliva, or the like of the subject) from the subject, analyzes the predetermined sample using a predetermined technique (e.g., mass spectrometry), and measures subject metabolite data of a plurality of metabolites contained in the predetermined sample. The measurement system 200 outputs the measured subject metabolite data to the calculation system 100.

When the calculation system 100 and the measurement system 200 are not communicatively connected via a network such as the Internet and exchange information via an interface such as a USB, the measurement system 200 may output the measured subject metabolite data to the calculation system 100 via an interface such as a USB.

For example, the measurement system 200 measures subject metabolite data of a plurality of metabolites included in correspondence data described below.

The output destination system 300 is an information processing system of the output destination to which the calculation system 100 outputs scores (disease scores and overall scores) and messages.

The output destination system 300 may be a medical institution system used by a medical institution (in particular, a physician or the like). That is, the calculation system 100 may notify a physician of scores (disease scores and overall score) and messages by outputting the scores (the disease scores and the overall scores) and the messages to the medical institution system.

Also, the output destination system 300 may be an information processing device used by the subject. This allows the subject to easily check the scores (the disease scores and the overall score) and the message without involving a third party (e.g., a medical institution).

Although Figure 2 shows one measurement system 200 and one output destination system 300, a plurality of measurement systems 200 and a plurality of output destination systems 300 may be provided.

Details of the calculation system 100 are described. The calculation system 100 includes a storage unit 110, a correspondence data acquisition unit 120, a reference individual data group acquisition unit 130, a metabolite data acquisition unit 140, a metabolite data extraction unit 150, a score calculation unit 160, a score output unit 170, a message output unit 180, an object output unit 190, and a report output unit 195. Each unit shown in Figure 2 can be implemented by using a storage area or by a processor executing a program stored in a storage area, for example.

The storage unit 110 stores information to be processed in the calculation system 100. For example, the storage unit 110 may store correspondence data, a reference individual data group, subject metabolite data, disease scores, overall scores, and message templates, which will be described below.

The correspondence data acquisition unit 120 acquires correspondence data indicating a correspondence between metabolites and diseases. The correspondence data acquisition unit 120 stores the acquired correspondence data in the storage unit 110.

The diseases may include, for example, obesity/diabetes, allergic diseases (in particular, atopic dermatitis, food allergies, allergic rhinitis (including hay fever), bronchial asthma, and the like), intestinal diseases (in particular, constipation, enteritis, irritable bowel syndrome, and the like), cardiovascular diseases (in particular, heart disease, ischemic heart disease, and the like), and non-alcoholic steatohepatitis. The diseases are not limited to these examples.

The metabolites are preferably intestinal bacterial metabolites, and in particular may be fatty acid metabolites, short-chain fatty acids, amino acid metabolites, and the like. Fatty acid metabolites may be, for example, linoleic acid metabolites, and particularly include, for example, HYA (10-hydroxy-cis-12-octadecenoic acid), KetoA (10-oxo-cis-12-octadecenoic acid), and KetoC (10-oxo-trans-11-octadecenoic acid). Short-chain fatty acids include, for example, acetic acid, propionic acid, and butyric acid. Amino acid metabolites include trimethylamine, for example. The metabolites are not limited to these. The reference individual data group may be composed of any combination of fatty acid metabolites, short-chain fatty acids, and amino acid metabolites, and a necessary reference individual data group may be referred to according to the disease. The metabolite data of a subject may also be metabolite data in accordance with the reference individual data group, but it is appropriately measured and selected according to the disease.

The correspondence data is data that indicates the correspondence between a disease and at least one metabolite that is considered to influence the risk of contracting the disease (disease risk).

The correspondence between diseases and metabolites may be freely determined by the administrator of the calculation system 100, for example. In this case, the administrator of the calculation system 100 may determine the correspondence between diseases and metabolites by referring to a predetermined academic paper. In other words, the correspondence data may be data indicating the correspondence between a disease and a metabolite that is scientifically considered to influence the risk of contracting that disease (disease risk).

The correspondence data acquisition unit 120 may acquire correspondence data created by the administrator of the calculation system 100. Also, the correspondence data acquisition unit 120 may acquire correspondence data from an administrator terminal of the administrator of the calculation system 100, for example.

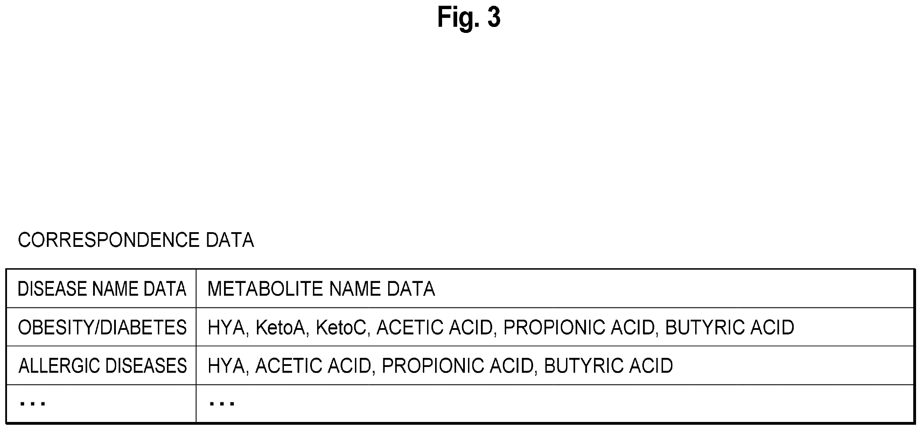

Figure 3 is a diagram showing an example of correspondence data stored in the storage unit 110. The correspondence data stored in the storage unit 110 may include disease name data and metabolite name data, for example. The disease name data is data indicating the disease names of the target diseases for which the calculation system 100 calculates disease scores, which will be described below. The metabolite name data is data that indicates the names of the metabolites corresponding to the disease name data.

The correspondence data stored in the storage unit 110 may include, instead of disease name data and metabolite name data, a disease ID that identifies a disease name and a metabolite ID that identifies a metabolite name, in association with each other.

The reference individual data group acquisition unit 130 acquires a reference individual data group that includes reference individual metabolite data of at least one metabolite of multiple reference individuals, and stores the acquired reference individual data group in the storage unit 110.

The reference individuals may include healthy individuals (for example, people who do not have a specific disease (in particular, a disease indicated by the disease name data included in the correspondence data or the like)). In other words, the reference individual data group may include, for example, metabolite data obtained by analyzing samples acquired from a plurality of healthy individuals. This allows the calculation system 100 to calculate a disease score of a subject by comparing the metabolite data of the subject with that of healthy individuals.

The reference individuals may also include, for example, non-healthy individuals (for example, a specific disease (in particular, people with a disease indicated by the disease name data included in the correspondence data or the like). In other words, the reference individual data group may include, for example, metabolite data obtained by analyzing samples acquired from a plurality of non-healthy individuals. This allows the calculation system 100 to calculate a disease score of a subject by comparing the metabolite data of the subject with that of non-healthy individuals.

Furthermore, the reference individuals may include one or more healthy individuals and one or more non-healthy individuals, for example. In other words, the reference individual data group may include metabolite data obtained by analyzing samples acquired from a plurality of healthy and non-healthy individuals, for example. This allows the calculation system 100 to calculate a disease score of the subject through comparison with metabolite data of healthy and non-healthy individuals.

When the reference individuals include healthy individuals and non-healthy individuals, the reference individual data group may include data (e.g., flags, labels) indicating whether the reference individual is a healthy individual or a non-healthy individual. This allows the calculation system 100 to determine whether the reference individual is a healthy individual or a non-healthy individual.

The reference individual data group includes at least one piece of reference individual metabolite data for each of reference individuals. At least one piece of reference individual metabolite data included in the reference individual data group may be reference individual metabolite data of a metabolite that corresponds to the metabolite name data included in the correspondence data. In this case, the metabolites include, for example, fatty acid metabolites, short-chain fatty acids, and amino acid metabolites, in particular, linoleic acid metabolites (e.g., HYA, KetoA, and KetoC), acetic acid, propionic acid, butyric acid, trimethylamine, and the like.

For example, reference individual metabolite data may be quantitative data on metabolites, and in particular, data relating to values of amount or concentration of metabolites. The units of the values indicated by the metabolite data may differ for each metabolite, and may be the amount of metabolite contained per gram of sample (mg or µg).

The reference individual data group acquisition unit 130 may acquire a reference individual data group from the administrator terminal, for example.

The reference individual data group acquisition unit 130 may also add subject metabolite data of a subject acquired by a metabolite data acquisition unit 140, which will be described below, to a reference individual data group and acquire the added reference individual data group. At this time, the reference individual data group acquisition unit 130 may determine whether the subject corresponds to a reference individual (for example, a healthy individual or a non-healthy individual), and add the metabolite data of this subject to the reference individual data group if the subject corresponds to a reference individual. This allows the calculation system 100 to expand the reference individual data group and improve the accuracy of the disease score.

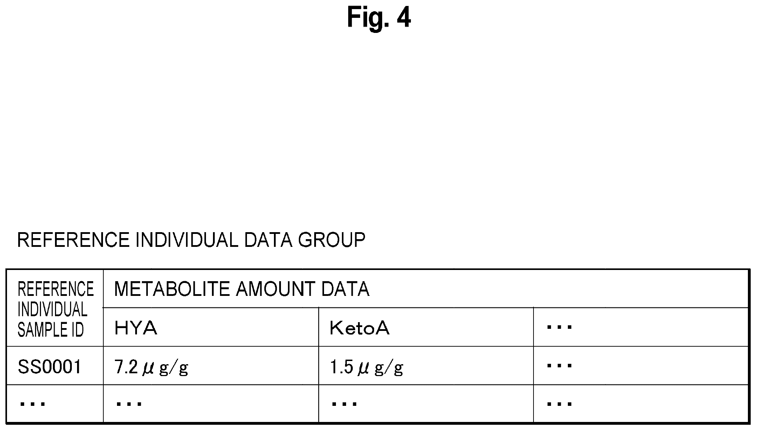

Figure 4 shows an example of a reference individual data group stored in the storage unit 110. The reference individual data group stored in the storage unit 110 includes reference individual sample ID and metabolite amount data. The reference individual sample ID is data that identifies the sample of a reference individual. The metabolite amount data included in the reference individual data group is data indicating the value of amount or concentration of each of a plurality of metabolites contained in the sample corresponding to the reference individual sample ID.

The metabolite data acquisition unit 140 acquires subject metabolite data relating to a plurality of metabolites in the subject and stores the acquired subject metabolite data in the storage unit 110.

For example, the subject metabolite data may be quantitative data of metabolites detected from the sample of the subject, and in particular, data relating to the values of amounts or concentrations of metabolites. The units of the values indicated by the metabolite data may differ for each metabolite, and may be the amount of metabolite contained per gram of sample (mg or µg).

The metabolite data acquisition unit 140 can acquire subject metabolite data from the measurement system 200, for example. When the calculation system 100 and the measurement system 200 are not communicatively connected via a network such as the Internet, and exchange information via an interface such as a USB, the metabolite data acquisition unit 140 may acquire subject metabolite data from a predetermined device, such as a USB, that stores the subject metabolite data.

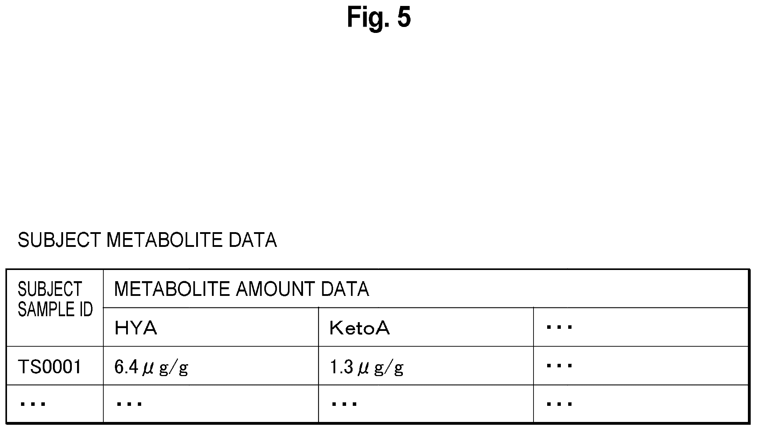

Figure 5 shows an example of subject metabolite data stored in the storage unit 110. The subject metabolite data stored in the storage unit 110 may include subject sample ID and metabolite amount data, for example. The subject sample ID is data that identifies the sample of a subject. The metabolite amount data included in the subject metabolite data is data indicating the value of amount or concentration of each of a plurality of metabolites contained in the sample corresponding to the sample ID.

The metabolite data extraction unit 150 extracts, from the acquired subject metabolite data, subject metabolite data of at least one metabolite corresponding to a target disease for which a score is to be calculated, on the basis of correspondence between metabolites and diseases.

Specifically, a case in which the target disease is "obesity/diabetes" is described. First, the metabolite data extraction unit 150 identifies the metabolites "HYA, KetoA, KetoC, acetic acid, propionic acid, butyric acid" corresponding to the disease "obesity/diabetes" on the basis of the correspondence data. Then, the metabolite data extraction unit 150 extracts the subject metabolite data of the metabolites "HYA, KetoA, KetoC, acetic acid, propionic acid, and butyric acid" from the acquired subject metabolite data.

Furthermore, when the target disease includes a plurality of target diseases, the metabolite data extraction unit 150 can extract subject metabolite data for each of the plurality of target diseases. Specifically, when the target diseases are, for example, the diseases "obesity/diabetes" and "allergic diseases," the metabolite data extraction unit 150 extracts subject metabolite data of the metabolites "HYA, KetoA, KetoC, acetic acid, propionic acid, and butyric acid" for the disease "obesity/diabetes," and extracts subject metabolite data of the metabolites "HYA, acetic acid, propionic acid, and butyric acid" for the disease "allergic diseases," on the basis of the correspondence data.

The score calculation unit 160 calculates a disease score relating to the risk of the target disease in the subject on the basis of the statistical association between the extracted subject metabolite data and the reference individual data group, and stores the calculated disease score in the storage unit 110.

When the target disease includes a plurality of target diseases, the score calculation unit 160 can calculate a disease score for each of the plurality of target diseases on the basis of the subject metabolite data extracted for each of the plurality of target diseases. Specifically, when the target diseases are, for example, the diseases "obesity/diabetes" and "allergic diseases," the score calculation unit 160 can calculate a disease score for the disease "obesity/diabetes" on the basis of the subject metabolite data of the metabolites "HYA, KetoA, KetoC, acetic acid, propionic acid, and butyric acid" corresponding to the disease "obesity/diabetes," and calculate a disease score for the disease "allergic diseases" on the basis of the subject metabolite data of the metabolites "HYA, acetic acid, propionic acid, and butyric acid" corresponding to the disease "allergic diseases" on the basis of the correspondence data.

The specific calculation method by the score calculation unit 160 is described. The score calculation unit 160 can calculate a disease score on the basis of statistical association (including, for example, a rank in the distribution of a data group), for example. Specifically, on the basis of the extracted subject metabolite data and the reference individual data group, a disease score can be calculated according to the rank of the subject having the extracted subject metabolite data in the distribution of the reference individual data group.

More specifically, when the extracted subject metabolite data is in the top 20% of the reference individual data group, the score calculation unit 160 can calculate the disease score as 80 points (that is, a score equivalent to 80% of the maximum score of 100 points). At this time, when a plurality of pieces of subject metabolite data are extracted for a single target disease, the score calculation unit 160 may calculate the average value of the scores (metabolite scores) calculated on the basis of each of the plurality of pieces of subject metabolite data as the disease score for that target disease, for example.

In this case, the score calculation unit 160 may calculate the disease score by assigning a predetermined weight to each of a plurality of metabolite scores. This allows the calculation system 100 to calculate a disease score in which a specific metabolite is given greater consideration. More specifically, for example, among a plurality of pieces of subject metabolite data, a higher weight is assigned to a metabolite having a greater influence on the risk of the target disease to calculate a disease score. The weighting may be different for each target disease.

As described above, the calculation system 100 calculates the disease score using a statistical association, in particular, a rank in a distribution or the like. This calculation method allows the calculation system 100 to calculate a disease score on the basis of both metabolite data of metabolites that are evaluated as having a lower disease risk when present in larger amounts, and metabolite data of metabolites that are evaluated as having a lower disease risk when present in smaller amounts. Furthermore, while a plurality of metabolites differ in the level (scale) of amount or concentration in a living body, this calculation method can evaluate the amounts of metabolites of the subject and reference individuals relatively rather than absolutely. Thus, a disease score can be calculated simply by evaluating a plurality of metabolites having different levels of amount or concentration.

The score calculation unit 160 can also calculate an overall score relating to the health status of the subject on the basis of a plurality of disease scores. For example, the score calculation unit 160 may calculate an average value of a plurality of disease scores as an overall score.

At this time, the score calculation unit 160 may calculate an overall score by assigning a predetermined weight to each of the plurality of disease scores. This allows the calculation system 100 to calculate an overall score in which a specific target disease is given greater consideration. More specifically, for example, among a plurality of target diseases, a higher weight is assigned to a disease having a greater influence on the health status of the subject to calculate an overall score.

Also, the score calculated by the score calculation unit 160 may be a score out of 100 points, but it is not limited to this and may be a five-level evaluation score, for example.

The score output unit 170 outputs a disease score. The score output unit 170 also outputs an overall score.

The score output unit 170 can output a disease score and an overall score to the output destination system 300, for example. This allows users of the output destination system 300 (for example, medical institutions, subjects, and the like) to obtain disease scores and overall scores. Disease scores and overall scores may be provided individually, or together with information representing other conditions of the subject. For example, they may be viewable on the output destination system 300, or they may be viewable as a list on paper or a sheet along with other information.

The message output unit 180 outputs, on the basis of a plurality of message templates that are set in advance and are in accordance with disease scores, a message for the subject including content relating to a risk of the target disease in the subject corresponding to the calculated disease score.

The calculation system 100 stores, in advance, a plurality of message templates, which include content relating to a risk of the target disease in the subject in accordance with disease scores, in the storage unit 110. The message output unit 180 refers to a plurality of message templates stored in the storage unit 110 and outputs a message corresponding to the calculated disease score. The message output processing by the message output unit 180 will be described below.

A plurality of message templates are stored in the storage unit 110 in advance. The message templates are set in advance, for example, by the administrator of the calculation system 100 and stored in the storage unit 110.

For example, the message may include content indicating that the disease risk is "high," "slightly high," "average," "slightly low," or "low" according to the disease score for each target disease. This allows the subject or the medical institution to understand the disease risk of the subject for each target disease.

Specifically, the plurality of message templates in accordance with disease scores for each target disease may be set such that a message indicating that the disease risk is high (for example, "Your risk of allergic disease has a tendency to be high.") is output when the disease score is less than 20 points, a message indicating that the disease risk is slightly high (for example, "Your risk of allergic disease has a tendency to be slightly high.") is output when the disease score is 20 points or more and less than 40 points, a message indicating that the disease risk is average (for example, "Your risk of allergic disease is average.") is output when the disease score is 40 points or more and less than 60 points, a message indicating that the disease risk is slightly low (for example, "Your risk of allergic disease has a tendency to be slightly low.") is output when the disease score is 60 points or more and less than 80 points, and a message indicating that the disease risk is low (for example, "Your risk of allergic disease has a tendency to be low.") is output when the disease score is 80 points or more.

Also, a message may be set for each metabolite, for example. In this case, messages for each metabolite may be set in accordance with the magnitude relationship between the metabolite data (e.g., the amount or concentration of the metabolite indicated by the metabolite data) and a predetermined threshold, for example. The predetermined threshold may be, for example, a statistical value (for example, a median value, an average value, or the like) of reference individual metabolite data corresponding to the metabolite included in the reference individual data group. This allows the subject or the medical institution to understand, for each metabolite, a state of the subject in comparison with reference individuals.

Specifically, the message templates in accordance with the magnitude relationship between metabolite data and a predetermined threshold may be set such that a message "Your metabolite HYA is higher than that of the reference individual data group". is output when the metabolite amount of the metabolite HYA indicated by the subject metabolite data is greater than the statistical value (for example, a median value, an average value, or the like) of the metabolite amount of the metabolite HYA indicated by the reference individual metabolite data included in the reference individual data group, and that a message "Your metabolite HYA is lower than that of the reference individual data group". is output when the metabolite amount of the metabolite HYA indicated by the subject metabolite data is less than the statistical value (for example, a median value, an average value, or the like) of the metabolite amount of the metabolite HYA indicated by the reference individual metabolite data included in the reference individual data group.

In addition, a message for each metabolite may be set according to a predetermined range of values to which the metabolite score corresponding to that metabolite belongs (for example, 80 points or more, 60 points or more and less than 80 points, or the like). In this case, the message output unit 180 extracts and outputs a corresponding message according to a predetermined range of values to which the metabolite score belongs.

Furthermore, when a message is set for each metabolite, the message may further include content relating to the metabolite (for example, content relating to the function of the metabolite). Specifically, the message may be a message relating to the function of the metabolite such as "HYA is known to have anti-allergic effects". or a message encouraging the improvement of lifestyle habits such as "Try to increase or supplement these metabolites". This allows the subject or the medical institution to understand the function of the metabolite, and can provide the subject with motivation to improve the health status, for example.

A message including content relating to a metabolite may be set, for example, according to the magnitude relationship between the metabolite data and a predetermined threshold, or may be set according to a predetermined range of values to which the disease score corresponding to the metabolite belongs. In this case, a message including content relating to a metabolite may be output when the metabolite amount of the metabolite indicated by the subject metabolite data is less than or equal to a predetermined threshold, or when the disease score corresponding to the metabolite is less than or equal to a predetermined value (e.g., 50 points). This allows the calculation system 100 to output a message that provides motivation to improve the health status for a subject whose metabolite amount belongs to a lower level in the distribution of the reference individual data group, for example.

Also, the message may be a message corresponding to the overall score. For example, the message in accordance with the overall score may be a message indicating the overall score, such as "Your overall score is 80 points.", a message relating to a metabolite amount, such as "You have a very high metabolite amount.", a message relating to the position in the distribution of the reference individual data group, such as "You are at the highest rank among all subjects.", and a message relating to the health status of the subject, such as "Your current lifestyle and dietary habits appear to be very good."

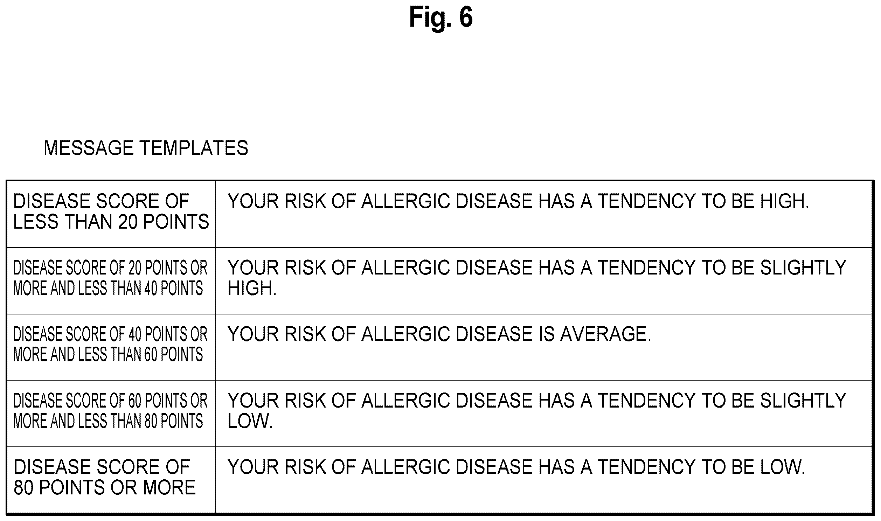

Figure 6 shows an example of message templates stored in the storage unit 110. The example message templates shown in Figure 6 are examples of a plurality of message templates in accordance with disease scores. As shown in Figure 6, the multiple message templates in accordance with disease scores may indicate whether the disease risk is "high," "slightly high," "average," "slightly low," or "low" in accordance with the disease score for each target disease, for example.

Message output processing by the message output unit 180 is described using a specific example. The message output unit 180 outputs, on the basis of a plurality of message templates that are set in advance and are in accordance with disease scores, a message for the subject including content relating to a risk of the target disease in the subject corresponding to the calculated disease score.

In this case, first, the message output unit 180 extracts, on the basis of the disease score, a message corresponding to a predetermined range of values to which the disease score belongs. More specifically, when the disease score is 80 points, the message output unit 180 extracts at least one message that is set to be output when the disease score is 80 points (that is, the message "Your risk of allergic disease has a tendency to be low.", for example).

The message output unit 180 then outputs at least one extracted message.

Thus, the message output unit 180 can output a message in accordance with the disease score (for example, "Your risk of allergic disease has a tendency to be low.").

The message output unit 180 can also output, on the basis of a plurality of message templates in accordance with the magnitude relationship between metabolite data and a predetermined threshold, a message further including content that relates to at least one metabolite and corresponds to the magnitude relationship between the extracted subject metabolite data and the predetermined threshold.

In this case, the message output unit 180 identifies the magnitude relationship between the metabolite amount indicated by metabolite data of a certain metabolite A and a predetermined threshold (for example, a statistical value (for example, a median value, an average value, or the like) of the metabolite amount indicated by the metabolite data of the metabolite A included in the reference individual data group). Then, the message output unit 180 extracts at least one message according to the identified magnitude relationship.

The message output unit 180 then outputs at least one extracted message template as a message.

This allows the message output unit 180 to output, for example, a different message for each metabolite (for example, "Your metabolite HYA is higher than that of the reference individual data group". and "Your metabolite HYA is lower than that of the reference individual data group.").

The message output unit 180 can also output a message including content relating to at least one metabolite corresponding to a statistical association between the extracted subject metabolite data and the reference individual data group, on the basis of a plurality of message templates in accordance with the statistical association.

In this case, first, the message output unit 180 extracts at least one message in accordance with a predetermined range of values to which the metabolite score corresponding to the metabolite belongs, on the basis of the metabolite score and the message templates.

The message output unit 180 then outputs at least one extracted message template as a message.

This allows the message output unit 180 to output, for example, a different message for each metabolite (for example, "Your metabolite HYA is higher than that of the reference individual data group". and "Your metabolite HYA is lower than that of the reference individual data group").

The message output unit 180 can also output messages in accordance with the combination of statistical associations between the extracted subject metabolite data and the reference individual data group, and the disease score.

Specifically, the message output unit 180 first extracts at least one message corresponding to the disease score and the metabolite score on the basis of the disease score, the metabolite score, and the message template. At this time, the message output unit 180 extracts, for example, a message that is in accordance with a predetermined range of values to which the disease score belongs, and is in accordance with a predetermined range of values to which the metabolite score corresponding to the metabolite belongs.

The message output unit 180 then outputs at least one extracted message template as a message.

Thus, for example, the message output unit 180 may output, for a subject whose disease score is less than a predetermined value, content relating to a metabolite with a score less than or equal to the predetermined value among a plurality of metabolites (for example, "HYA is known to have anti-allergic effects". and "Your metabolite HYA is lower than that of the reference individual data group."). In contrast, for a subject whose disease score is greater than or equal to the predetermined value, the message output unit 180 may output no message of content relating to metabolites.

The message output processing by the message output unit 180 described in this embodiment is merely an example, and the message output processing by the message output unit 180 is not limited to these examples.

Also, "disease score" may be appropriately read as "overall score", and the message output unit 180 may output a message in accordance with the overall score. Furthermore, message output processing based on at least one of the disease score and the metabolite score may output a message taking into account the overall score.

The message output unit 180 may combine at least one extracted message template and output the combined template as one or more messages. Furthermore, the message output unit 180 may combine a fixed message template, which is set in advance and independent of a score (at least one of a metabolite score, disease score, and overall score), with the extracted message template and output the combined template as one or more messages.

The object output unit 190 may output an object that displays a distribution of the reference individual data group and displays a portion of the object corresponding to the disease score in a different manner.

For example, the object output unit 190 may output, as an object, a frequency distribution that indicates a distribution of the reference individual data group.

The report output unit 195 can generate and output a report that includes an overall score, a disease score, a message, and an object. For example, the report may be a paper document or electronic data (e.g., portable document format (PDF)). Furthermore, the report may be displaying data for displaying this report on the output destination system 300 (for example, a display unit of the output destination system).

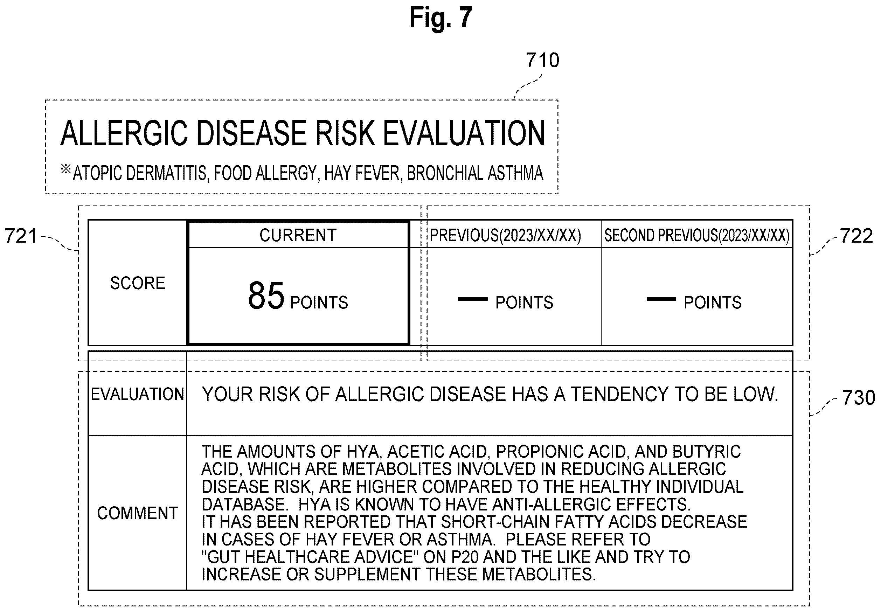

Figure 7 shows an example of a report output by the calculation system 100. The report shown in Figure 7 is a report including a disease score and a message, for example. For example, the report shown in Figure 7 includes a region 710 for displaying data relating to the target disease, a region 721 for displaying a disease score, a region 722 for displaying the previous and second previous disease scores, and a region 730 for displaying a message.

For example, the message displayed in region 730 may further include a region for displaying a summary of the message as "evaluation" and a region for displaying details of the message as "comment". For example, the message displayed in region 730 may include messages including content relating to the risk of the target disease in accordance with the disease score, and messages including content relating to at least one metabolite corresponding to the target disease.

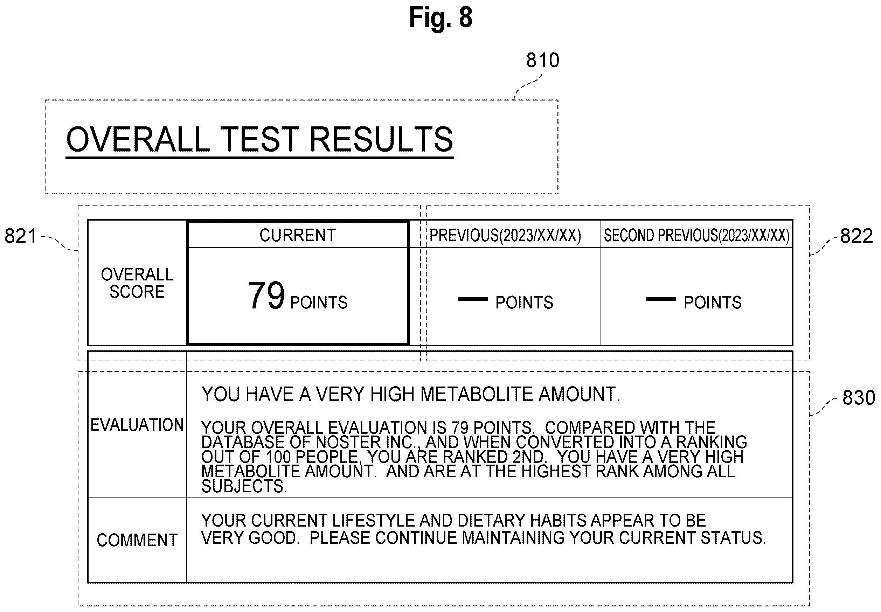

Figure 8 shows an example of a report output by the calculation system 100. The report shown in Figure 8 is a report that includes an overall score and a message, for example. The report shown in Figure 8 includes, for example, region 810 for displaying the report title, region 821 for displaying an overall score, region 822 for displaying the previous and second previous overall scores, and region 830 for displaying a message.

For example, the message displayed in region 830 may further include a region for displaying a summary of the message as "evaluation" and a region for displaying details of the message as "comment". The messages displayed in region 830 may include a message including content relating to the health status of the subject in accordance with the overall score, for example.

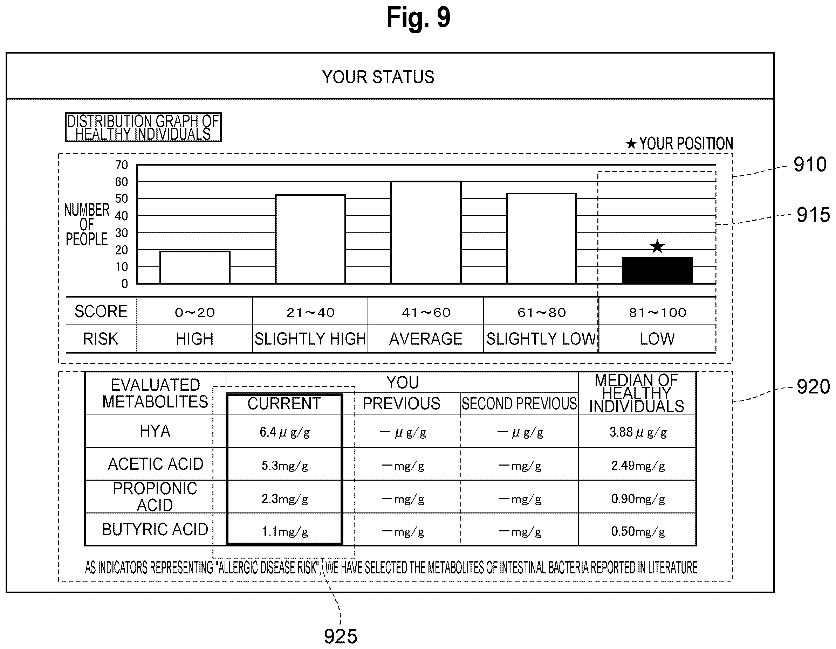

Figure 9 shows an example of a report output by the calculation system 100. The report shown in Figure 9 is a report including an object for displaying a distribution of the reference individual data group. For example, the report shown in Figure 9 may include region 910 for displaying an object representing a distribution of the reference individual data group, and region 920 for displaying the numerical values of the subject metabolite data and statistical values of the reference individual data group (for example, a median value, an average value, or the like).

Region 910 further includes region 915 that displays a portion of the object corresponding to the disease score in a different manner. Region 920 further includes region 925 for displaying numerical values of the subject metabolite data, and region 925 is displayed in a manner different from the other regions.

The different manner may include highlighting using a rectangle of a predetermined color, enclosing with a line of a predetermined color, displaying with text of a predetermined color, and displaying the text in bold or italics, but it is not limited to these.

The reports shown in Figures 7 to 9 are merely examples, and the layout of the report and the content displayed are not limited to these.

The report output unit 195 can output the report to a medical institution, for example. This allows the subject to receive a score calculation service using the calculation system 100 as part of a diagnosis performed at a medical institution upon request (for example, a periodically performed medical checkup).

When the score output unit 170 and the message output unit 180 output a score and a message to the output destination system 300, the output destination system 300 can generate a report that displays a score and a message based on the acquired score and message. This allows a user of the output destination system 300 (for example, a medical institution) to generate a report in a desired layout and format.

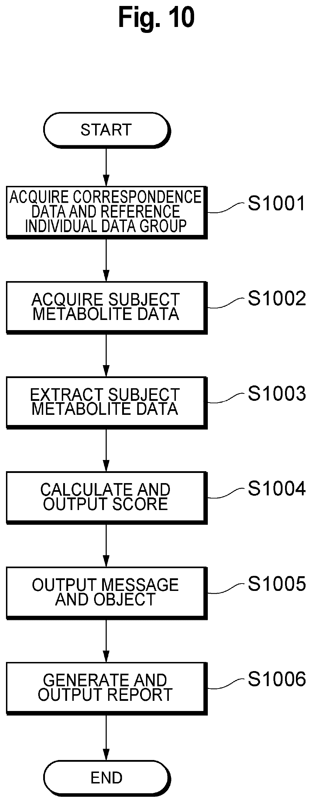

Figure 10 is a flowchart showing an example of processing by the calculation system 100.

First, the correspondence data acquisition unit 120 acquires correspondence data, and the reference individual data group acquisition unit 130 acquires reference individual data group (S1001). The metabolite data acquisition unit 140 acquires subject metabolite data of a plurality of metabolites in the subject (S1002). The metabolite data extraction unit 150 extracts the subject metabolite data of at least one metabolite from the subject metabolite data of multiple metabolites (S1003).

The score calculation unit 160 then calculates a disease score and an overall score, and the score output unit 170 outputs the disease score and the overall score (S1004). The message output unit 180 outputs a message in accordance with the score, and the object output unit 190 outputs an object that displays a distribution of the reference individual data group and displays a portion of the object corresponding to the disease score in a different manner (S1005). The report output unit 195 generates and outputs a report that displays the disease score, the overall score, the message, and the object (S1006).

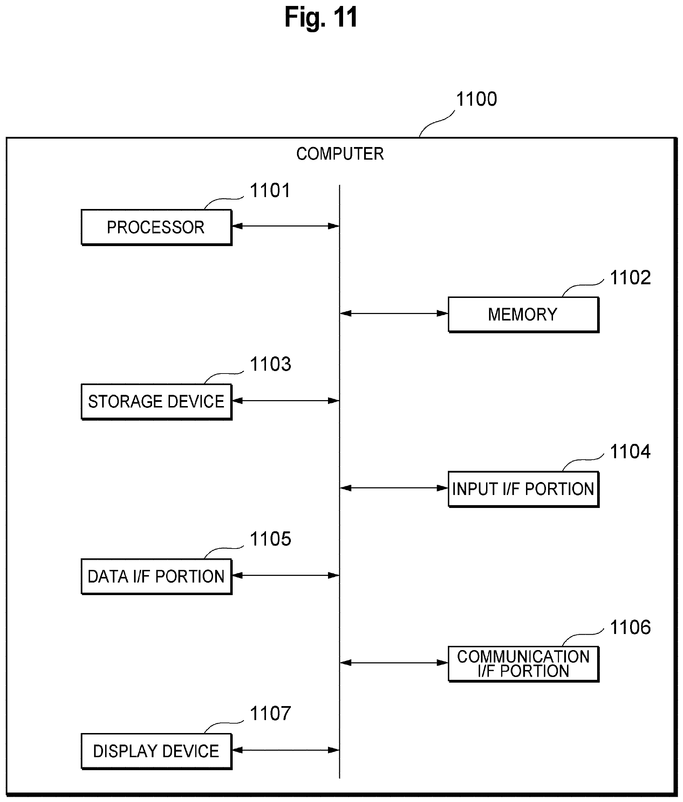

Referring to Figure 11, an example of a hardware configuration when the calculation system 100 is implemented by a computer 1100 is described. Figure 11 is a diagram illustrating an example of the hardware configuration of a computer 1100.

As shown in Figure 11, the computer 1100 includes, for example, a processor 1101, a memory 1102, a storage device 1103, an input I/F portion 1104, a data I/F portion 1105, a communication I/F portion 1106, and a display device 1107.

The computer 1100 may be, for example, a server computer, a personal computer (e.g., desktop, laptop, tablet, and the like), a media computer platform (e.g., cable, satellite set-top box, digital video recorder, and the like), a handheld computer device (e.g., PDA, email client, and the like), or any other type of computer or communications platform.

The processor 1101 is a control unit that controls various processes in the computer 1100 by executing a program stored in the memory 1102.

The memory 1102 is a storage medium such as random access memory (RAM). The memory 1102 temporarily stores the program code of the program executed by the processor 1101 and data required when the program is executed.

The storage device 1103 is a non-volatile storage medium such as a hard disk drive (HDD) or a flash memory. The storage device 1103 stores an operating system and various programs for implementing the above-mentioned components.

The input I/F portion 1104 is a device for receiving input from the user. For example, the input I/F portion 1104 may be a keyboard, a mouse, a touch panel, various sensors, a wearable device, or the like. The input I/F portion 1104 may be connected to the computer 1100 via an interface such as a USB.

The data I/F portion 1105 is a device for inputting data from outside the computer 1100. For example, the data I/F portion 1105 may be a drive device for reading data stored in various storage media. The data I/F portion 1105 may be provided outside the computer 1100. When the data I/F portion 1105 is provided outside the computer 1100, the data I/F portion 1105 is connected to the computer 1100 via an interface such as a USB.

The communication I/F portion 1106 is a device for performing data communication with external devices of the computer 1100 via a network such as the Internet, either by wire or wireless connection. The communication I/F portion 1106 may be provided outside the computer 1100. When the communication I/F portion 1106 is provided outside the computer 1100, the communication I/F portion 1106 is connected to the computer 1100 via an interface such as a USB.

The display device 1107 is a device for displaying various types of information. The display device 1107 is, for example, a liquid crystal display, an organic electro-luminescence (EL) display, a display of a wearable device, or the like. The display device 1107 may be provided externally to the computer 1100. When the display device 1107 is provided outside the computer 1100, the display device 1107 is connected to the computer 1100 via, for example, a display cable or the like. Also, when a touch panel is used as the input I/F portion 1104, the display device 1107 may be configured integrally with the input I/F portion 1104.

An embodiment of the present disclosure has been described above. The calculation system 100 can acquire subject metabolite data of a subject, extract subject metabolite data of at least one metabolite corresponding to a target disease, on the basis of the correspondence between metabolites and diseases, calculate a disease score of the subject on the basis of the statistical association between the extracted subject metabolite data and a reference individual data group, and output the disease score. This allows the calculation system 100 to calculate the risk of contracting a specific disease on the basis of metabolite data.

The calculation system 100 can also extract subject metabolite data of at least one metabolite for each of a plurality of target diseases, and calculate a disease score. This allows the calculation system 100 to calculate the risk of contracting a specific disease for each of the target diseases.

The calculation system 100 can also calculate and output an overall score on the basis of the disease score for each of the target diseases. This allows the calculation system 100 to evaluate the health status of a subject on the basis of the risk of contracting a specific disease.

The calculation system 100 can also calculate a disease score on the basis of the rank of the subject in the distribution of the reference individual data group. This allows the calculation system 100 to calculate a disease score by comparing the distribution of reference individual data group with the subject metabolite data.

The calculation system 100 can also output a message including content relating to the risk of a target disease in a subject corresponding to the disease score. This allows a subject or a medical institution, for example, to understand the risk of the target disease in the subject.

The calculation system 100 can also output a message including content relating to at least one metabolite that corresponds to the statistical association between the extracted metabolite data and the reference individual data group. This allows the subject and a medical institution, for example, to understand content relating to a metabolite for which the score is lower than that of reference individuals.

The calculation system 100 can also output messages in accordance with the statistical association between the extracted metabolite data and the reference individual data group, and the disease score. This allows a subject or a medical institution, for example, to understand the risk of the target disease in the subject.

The calculation system 100 can also output an object for displaying a distribution of the reference individual data group and displays a portion of the object corresponding to the disease score in a different manner. This allows the subject or a medical institution, for example, to intuitively understand the position of the disease score of the subject in the distribution of the reference individual data group.

It should be noted that the present embodiment is intended to facilitate understanding of the present disclosure and is not intended to limit the present disclosure. The present disclosure may be modified/improved without departing from the spirit thereof, and the present disclosure also includes equivalents thereof.

Furthermore, in this disclosure, "unit" does not simply mean a physical means, but also includes cases where the functions of the "unit" are implemented by software. Furthermore, the functions of one "unit" or device may be implemented by two or more physical means, devices, or software, and the functions of two or more "units" or devices may be implemented by one physical means, device, or software.

### Reference Signs List

- 100: Calculation system
- 110: Storage unit
- 120: Correspondence data acquisition unit
- 130: Reference individual data group acquisition unit
- 140: Metabolite data acquisition unit
- 150: Metabolite data extraction unit
- 160: Score calculation unit
- 170: Score output unit
- 180: Message output unit
- 190: Object output unit
- 195: Report output unit
- 200: Measurement system
- 300: Output destination system

## Claims

1. A calculation system comprising:
a metabolite data acquisition unit configured to acquire subject metabolite data relating to amounts or concentrations of a plurality of metabolites in a subject;
a metabolite data extraction unit configured to extract, from the subject metabolite data that have been acquired, subject metabolite data of at least one metabolite corresponding to a target disease for which a score is to be calculated, on the basis of correspondence between metabolites and diseases;
a score calculation unit configured to calculate a disease score relating to a risk of the target disease in the subject on the basis of statistical association between the subject metabolite data that have been extracted and a reference individual data group that is acquired in advance and includes reference individual metabolite data relating to an amount or concentration of the at least one metabolite in a plurality of reference individuals; and
a score output unit configured to output the disease score.

2. The calculation system according to claim 1, wherein
the target disease includes a plurality of target diseases,
the metabolite data extraction unit is configured to extract subject metabolite data of at least one metabolite for each of the plurality of target diseases, and
the score calculation unit is configured to calculate the disease score for each of the plurality of target diseases.

3. The calculation system according to claim 2, wherein
the score calculation unit is further configured to calculate an overall score relating to a health status of the subject on the basis of the disease score for each of the plurality of target diseases, and
the score output unit is configured to output the overall score.

4. The calculation system according to any one of claims 1 to 3, wherein
the statistical association includes a rank in a distribution of a data group, and
the score calculation unit is configured to calculate the disease score on the basis of a rank of the subject in a distribution of the reference individual data group.

5. The calculation system according to claim 1 or 2, further comprising a message output unit configured to output, on the basis of a plurality of message templates that are set in advance and that are in accordance with disease scores, a message for the subject including content relating to a risk of the target disease in the subject, the message corresponding to the calculated disease score.

6. The calculation system according to claim 5, wherein the message output unit is configured to generate, on the basis of a plurality of message templates that are set in advance and that are in accordance with a magnitude relationship between metabolite data and a predetermined threshold, the message further including content that relates to the at least one metabolite and corresponding to a magnitude relationship between the extracted subject metabolite data and the predetermined threshold.

7. The calculation system according to claim 6, wherein the message output unit is configured to output the message in accordance with a magnitude relationship between the extracted subject metabolite data and the predetermined threshold, and the disease score.

8. The calculation system according to claim 7, further comprising an object output unit configured to output an object for displaying in a different manner a distribution of the reference individual data group, the object displaying a portion of the object corresponding to the disease score.

9. A calculating method, comprising, by a computer:
acquiring subject metabolite data relating to a plurality of metabolites in a subject;
extracting, from the subject metabolite data that have been acquired, subject metabolite data of at least one metabolite corresponding to a target disease for which a score is to be calculated, on the basis of correspondence between metabolites and diseases;
calculating a disease score relating to a risk of the target disease in the subject on the basis of statistical association between the subject metabolite data that have been extracted and a reference individual data group that is acquired in advance and includes reference individual metabolite data of the at least one metabolite in a plurality of reference individuals; and
outputting the disease score.

10. A calculation program for causing a computer to implement:
a metabolite data acquisition unit configured to acquire subject metabolite data relating to a plurality of metabolites in a subject;
a metabolite data extraction unit configured to extract, from the subject metabolite data that have been acquired, subject metabolite data of at least one metabolite corresponding to a target disease for which a score is to be calculated, on the basis of correspondence between metabolites and diseases;
a score calculation unit configured to calculate a disease score relating to a risk of the target disease in the subject on the basis of statistical association between the subject metabolite data that have been extracted and a reference individual data group that is acquired in advance and includes reference individual metabolite data of the at least one metabolite in a plurality of reference individuals; and
a score output unit configured to output the disease score.
